# FASCICULE DE BREVET EUROPEEN

(11) **EP 3 360 004 B1**
(45) Date de publication et mention de la délivrance du brevet: **15.01.2025**
(21) Numéro de dépôt: 16787508.7
(22) Date de dépôt: 06.10.2016
(51) Int. Cl.: G02C 7/02, G02C 13/00, A61B 3/10

(54) **PROCEDE DE CONTROLE DE LA FAISABILITE D'UNE PAIRE DE LUNETTES**
VERFAHREN ZUR KONTROLLE DER MACHBARKEIT EINER BRILLENLINSEN-PAAR
PROCESS FOR CONTROLLING THE FEASIBILITY OF A PAIR OF SPECTACLES

(30) Priorité: 09.10.2015 FR 1559651
(43) Date de publication de la demande: 15.08.2018
(73) Titulaire: Essilor International, 94220 Charenton-le-Pont (FR)
(72) Inventeur: HOLVOET VERMAUT, Benoit, 94220 Charenton-le-Pont (FR); MARTIN, Luc, 94220 Charenton-le-Pont (FR)
(74) Mandataire: Jacobacci Coralis Harle
(86) Numéro de dépôt international: PCT/FR2016/052584
(87) Numéro de publication internationale: WO 2017/060639

(56) Documents cités:
- EP-A1- 1 468 649
- FR-A1- 2 885 231
- FR-A1- 2 910 647
- FR-A1- 3 016 052
- JP-A- 2007 240 553
- US-A1- 2003 123 026
- US-A1- 2013 231 941
- US-A1- 2013 335 700

## Description

### DOMAINE TECHNIQUE AUQUEL SE RAPPORTE L'INVENTION

La présente invention concerne de manière générale le domaine de la lunetterie.

Elle concerne plus particulièrement un procédé de contrôle de la faisabilité d'une paire de lunettes, c'est-à-dire permettant de vérifier *a priori* la possibilité d'assembler une paire de lunettes.

### ARRIERE-PLAN TECHNOLOGIQUE

La partie technique du métier de l'opticien consiste à monter une paire de lentilles ophtalmiques correctrices sur une monture de lunettes sélectionnée par un porteur.

Ce montage se décompose en trois opérations principales :
- l'acquisition de la géométrie d'un profil longitudinal représentatif de la forme du contour de l'un des entourages de la monture de lunettes sélectionnée,
- le centrage de la lentille ophtalmique considérée qui consiste à positionner et à orienter convenablement ce profil longitudinal sur la lentille de manière qu'une fois la lentille détourée suivant ce profil longitudinal puis montée dans sa monture, elle soit correctement positionnée par rapport à l'oeil correspondant du porteur afin qu'elle puisse exercer au mieux la fonction optique pour laquelle elle a été conçue, puis
- le détourage de la lentille qui consiste à usiner son contour à la forme du profil longitudinal.

A titre d'exemple, on peut s'intéresser plus particulièrement au cas des montures de lunettes cerclées. Dans ce type de montures, l'entourage (ou « cercle ») est conçu pour entourer l'ensemble de la périphérie de la lentille. Il comporte, pour le maintien de la lentille, une rainure intérieure appelée drageoir.

L'opération d'acquisition consiste alors à palper le contour du drageoir pour en déterminer la forme. L'opération de détourage consiste quant à elle à détourer et biseauter le chant de la lentille pour y faire apparaître une nervure d'emboîtement pouvant s'emboîter dans le drageoir.

Il arrive malheureusement que certaines montures de lunettes ne soient pas compatibles avec toutes les lentilles ophtalmiques et que l'assemblage ne soit pas réalisable.

Un exemple illustrant bien ce problème est celui des lentilles ophtalmiques très courbées, qui ne peuvent pas s'emboîter dans les cercles des montures de lunettes très peu courbées.

Un autre exemple est celui des personnes dont les yeux sont très proches l'un de l'autre, pour lesquelles le point pupillaire (c'est-à-dire le point de la lentille en face duquel se trouvera l'oeil du porteur) sera très décentré par rapport au centre de l'entourage de la monture de lunettes. Dans ce cas, il sera nécessaire d'utiliser une lentille ophtalmique de grand diamètre. Or, pour certaines prescriptions de puissances, le diamètre disponible de lentilles est réduit (les lentilles présentaient sinon des épaisseurs au bord ou au centre trop importantes). Une impossibilité de montage peut ainsi survenir.

Il peut arriver que l'opticien ne prenne conscience de cette impossibilité de montage de la lentille ophtalmique dans la monture de lunettes qu'au moment de l'assemblage de la paire de lunettes. Dans cette éventualité, l'opticien est alors forcé de rappeler le client pour lui demander de choisir une autre monture.

S'il dispose de suffisamment d'expérience, l'opticien peut éviter ce problème en ne proposant, lorsque le porteur vient choisir sa monture de lunettes, qu'un échantillon réduit de montures pour lesquelles il estime qu'il sera possible de monter des lentilles ophtalmiques (compte tenu de la prescription du porteur et de la forme de son visage). Cet échantillon est alors généralement plus réduit qu'il ne le devrait puisque l'opticien ne peut pas estimer exactement quelles sont les montures de lunettes qui seront utilisables. L'inconvénient est alors que le porteur se voit proposer un nombre très restreint de montures de lunettes. Un document d'art antérieur pertinent est la demande de brevet FR 2 885 231 A1.

### OBJET DE L'INVENTION

Afin de remédier aux inconvénients précités de l'état de la technique, la présente invention propose une méthode permettant de déterminer de manière automatique dans quelle mesure une paire de lunettes pourra être assemblée.

On propose selon l'invention un procédé de contrôle tel que défini dans la revendication 1.

Ainsi, grâce à l'invention, pour chaque monture de lunettes, on va pouvoir savoir dans quelle mesure cette monture pourra accueillir les lentilles ophtalmiques d'un porteur, en déterminant simplement les valeurs des premier et second paramètres et en vérifiant qu'ils appartiennent aux gammes de valeurs pour lesquelles la paire de lunettes a été qualifiée de « faisable ».

D'autres caractéristiques avantageuses et non limitatives du procédé de contrôle sont les suivantes :
- lesdits premier et second paramètres sont des paramètres ophtalmiques, morphologiques ou géométrico-morphologiques caractérisant des porteurs de lunettes ;
- lesdits premier et second paramètres sont choisis dans la liste suivante : puissance de réfringence sphérique, puissance de réfringence cylindrique, hauteur pupillaire, écart ou demi-écart pupillaire ;
- à l'étape a), on acquiert une gamme de valeurs de deux premiers paramètres, et, à l'étape c), on vérifie, pour un nombre caractéristiques de valeurs de chaque second paramètre, que la paire de lunettes est faisable quelles que soient les valeurs des deux premiers paramètres comprises dans leur gamme ;
- à l'étape b), on acquiert une gamme de valeurs de deux seconds paramètres, et, à l'étape c), on vérifie, pour un nombre caractéristiques de couples de valeurs des seconds paramètres, que la paire de lunettes est faisable quelle que soit la valeur du premier paramètre comprise dans sa gamme ;
- à l'étape c), on évalue un niveau de faisabilité de la paire de lunettes, ledit niveau de faisabilité pouvant prendre au moins trois valeurs distinctes ;
- il est prévu, préalablement à l'étape a), une étape d'acquisition d'une donnée de courbure de la monture de lunettes et, à l'étape a), le premier paramètre étant une puissance de réfringence sphérique ou cylindrique, la gamme de valeurs du premier paramètre est choisie en fonction de ladite donnée de courbure de la monture de lunettes ;
- considérant une sous-gamme de la gamme de valeurs de chaque second paramètre dans laquelle la paire de lunettes n'est pas faisable, il est prévu une étape d) consistant à vérifier, pour un nombre caractéristiques de valeurs de chaque premier paramètre, que la paire de lunettes est faisable quelle que soit la valeur de chaque second paramètre compris dans ladite sous-gamme ;
- considérant une sous-gamme de la gamme de valeurs de chaque second paramètre dans laquelle la paire de lunettes est faisable et une sur-gamme englobant la gamme de valeurs du premier paramètre, il est prévu une étape d) consistant à vérifier, pour un nombre caractéristiques de valeurs du premier paramètre compris dans ladite sur-gamme, que la paire de lunettes est faisable quelle que soit la valeur de chaque second paramètre compris dans ladite sous-gamme.

L'invention telle que revendiquée concerne aussi une méthode de classement de montures de lunettes selon la revendication 9, au moyen d'une base de données comprenant des enregistrements chacun associés à une monture de lunettes, comprenant une opération de calcul au cours de laquelle on met en oeuvre le procédé de contrôle selon l'une des revendications précédentes pour une monture de lunettes identifiée, et une opération de stockage au cours de laquelle on enregistre, dans un nouvel enregistrement de la base de données associé à ladite monture de lunettes identifiée, la gamme de valeurs de chaque premier paramètre ainsi qu'une gamme de valeurs de chaque second paramètre dans l'ensemble de laquelle la paire de lunettes est faisable.

Préférentiellement, il est prévu une opération d'acquisition d'une première valeur particulière du premier paramètre et d'une seconde valeur particulière du second paramètre, ces première et seconde valeurs correspondant à un porteur de lunettes donné, et une opération de sélection des enregistrements de ladite base de données dans lesquels les gammes mémorisées comprennent lesdites première et seconde valeurs particulières.

Un exemple non revendiqué concerne également un catalogue de montures de lunettes disponibles pour fabriquer des paires de lunettes, comprenant, pour chaque monture de lunettes :
- un identifiant de la monture de lunettes,
- une gamme de valeurs d'au moins un premier paramètre relatif à un porteur de lunettes,
- une gamme de valeurs d'au moins un second paramètre relatif à un porteur de lunette, et qui est distinct de chaque premier paramètre,
lesdites gammes de valeurs étant définies de telle manière qu'il soit possible de fabriquer une paire de lunettes avec ladite monture de lunettes quelles que soient les valeurs des premier et second paramètres comprises dans leurs gammes.

Un autre exemple non revendiqué concerne enfin un indicateur de faisabilité d'une paire de lunettes au moyen d'une monture de lunettes identifiée, calculé en fonction de :
- une gamme de valeurs d'au moins un premier paramètre relatif à un porteur de lunettes,
- une gamme de valeurs d'au moins un second paramètre relatif à un porteur de lunette, et qui est distinct de chaque premier paramètre,
lesdites gammes de valeurs étant définies de telle manière qu'il soit possible de fabriquer une paire de lunettes avec ladite monture de lunettes quelles que soient les valeurs des premier et second paramètres comprises dans leurs gammes.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention qui est définie par les revendications, et comment elle peut être réalisée.

Sur les dessins annexés :
- la figure 1 est un organigramme illustrant les étapes d'un algorithme permettant la mise en oeuvre d'un premier mode de réalisation du procédé de contrôle conforme à l'invention ;
- la figure 2 représente deux graphiques illustrant les gammes des premiers et seconds paramètres utilisés dans le cadre du procédé de contrôle de la figure 1 ;
- la figure 3 est un organigramme illustrant les étapes d'un algorithme permettant la mise en oeuvre d'un second mode de réalisation du procédé de contrôle conforme à l'invention ;
- la figure 4 représente deux graphiques illustrant les gammes des premiers et seconds paramètres utilisés dans le cadre du procédé de contrôle de la figure 3 ;
- la figure 5 représente deux graphiques illustrant les gammes des premiers et seconds paramètres utilisés dans le cadre d'un troisième mode de réalisation d'un procédé de contrôle conforme à l'invention.

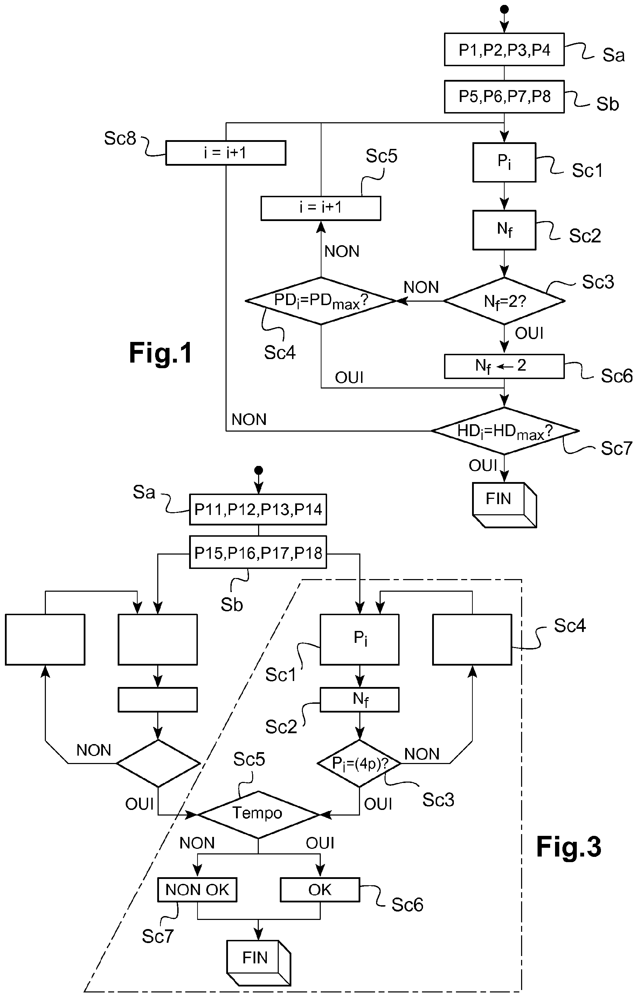

Sur les figures 1 et 3, on a représenté deux organigrammes illustrant les étapes permettant de mettre en oeuvre deux modes de réalisation d'un procédé de contrôle de la faisabilité d'une paire de lunettes.

Classiquement, une paire de lunettes comporte une monture de lunettes, et deux lentilles ophtalmiques. On dit d'une paire de lunettes qu'elle est faisable (ou « assemblable ») lorsqu'il est possible de monter les deux lentilles ophtalmiques dans la monture de lunettes, de telle manière que l'ensemble soit solide et esthétique.

L'objectif du procédé de contrôle est de vérifier a priori quelles lentilles ophtalmiques pourraient être montées dans une monture de lunettes préalablement identifiée, et quelles lentilles ophtalmiques ne pourraient pas y être montées (sauf éventuellement à tolérer des défauts dans l'assemblage de la monture et des lentilles).

Ce procédé est mis en oeuvre par tout type de dispositif électronique et/ou informatique.

Ici, il sera mis en oeuvre par un calculateur tel qu'un ordinateur, comportant un processeur (CPU), une mémoire vive (RAM), une mémoire morte (ROM), et différentes interfaces d'entrée et de sortie.

Grâce à ses interfaces d'entrée, le calculateur est adapté à recevoir des signaux d'entrée provenant de différents moyens d'acquisition. Un moyen d'acquisition pourra être un écran tactile ou un clavier, permettant à l'utilisateur de saisir des informations. Un autre moyen d'acquisition pourra être un dispositif de détermination de la forme de la monture de lunettes. Les signaux d'entrée pourront alors être relatifs à la forme de la monture de lunettes et aux informations saisies par l'utilisateur.

La mémoire morte mémorise quant à elle des données utilisées dans le cadre du procédé de contrôle décrit ci-dessous.

Comme cela apparaîtra bien dans la suite de cet exposé, elle mémorise notamment un registre de base de données, qui comporte plusieurs enregistrements associant chacun, à une référence de monture de lunettes, des gammes de valeurs indiquant dans quelle mesure une lentille ophtalmique est « montable » dans cette monture de lunettes.

Elle mémorise aussi une application informatique, constituée de programmes d'ordinateur comprenant des instructions dont l'exécution par le processeur permet la mise en oeuvre par le calculateur du procédé décrit ci-après.

La monture de lunettes utilisée dans le cadre du procédé décrit ci-après est « identifiée » en ce sens que le procédé de contrôle s'applique à une monture de lunettes dont les caractéristiques principales sont connues.

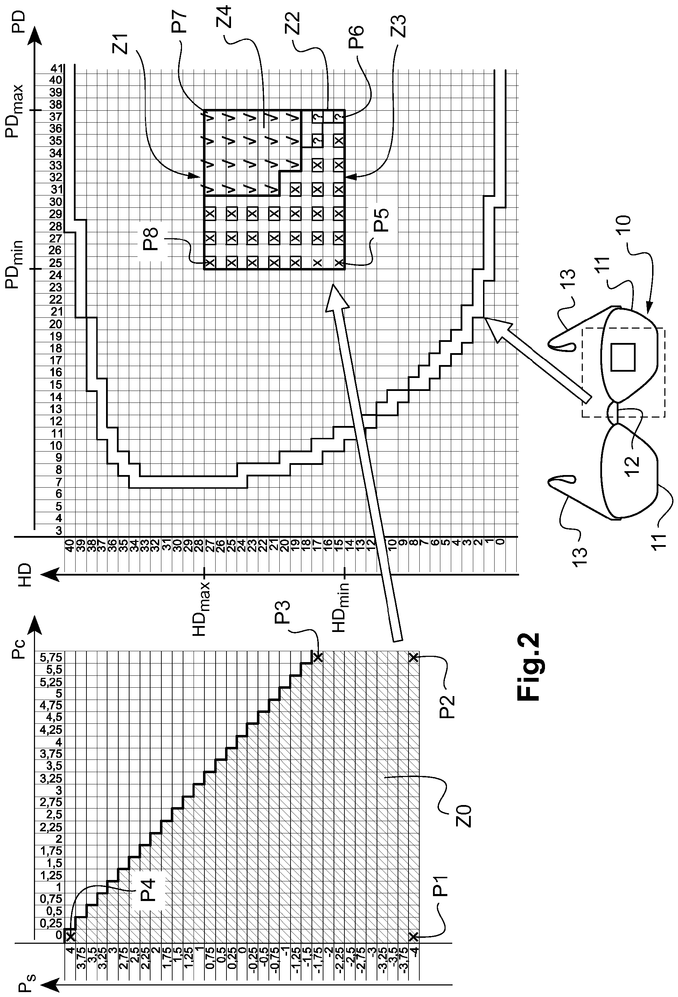

Cette monture de lunettes pourra être de tout type. Telle qu'elle est représentée sur le bas de la figure 2, il s'agit d'une monture de lunettes cerclée, c'est-à-dire d'une monture de lunettes comportant deux entourages 11 rigides (ou « cercles ») reliés entre eux par un pontet 12 et portant chacun une branche 13. Chacun de ces deux entourages 11 présente intérieurement une rainure (ou « drageoir ») dans laquelle est destinée à s'emboîter une nervure (ou « biseau ») courant le long du chant de la lentille ophtalmique.

Ici, parmi les caractéristiques principales de la monture de lunettes 10, on connaît au moins l'écart entre les deux entourages 11 et la forme du drageoir de chaque entourage 11.

En variante, il pourrait s'agir d'un autre type de monture de lunettes. A titre d'exemple non limitatif, il pourrait s'agir d'une monture semi-cerclée (dont chaque entourage comporte une arcade supérieure rigide et un fil qui est prévu pour passer sous la lentille) ou d'une monture percée (le pontet et les branches de la monture comportant alors des moyens de fixation au travers de trous prévus dans les lentilles).

Chaque lentille ophtalmique susceptible d'être montée dans la monture de lunettes 10 peut être définie par des caractéristiques géométriques (diamètre initial, épaisseur au centre, ...) et par des caractéristiques optiques.

Ces caractéristiques optiques définissent le pouvoir de correction optique. Elles s'expriment sous la forme de propriétés de réfringence sphérique, cylindrique et prismatique.

On comprend qu'une telle définition optique revêt une portée plus générale qu'une définition purement surfacique : elle définit dans sa globalité l'effet de réfringence de la lentille sur un rayon lumineux incident, résultant de la somme algébrique de la réfringence opérée successivement sur les faces avant et arrière de la lentille.

Parmi ces propriétés de réfringence, on définit tout d'abord la « puissance de réfringence sphérique » d'une lentille pour un faisceau de rayons incident traversant cette lentille (également appelée puissance totale ou puissance réfringente ou puissance de focalisation ou puissance optique sphérique), comme la grandeur qui caractérise et quantifie le premier effet de réfringence sphérique (effet « loupe ») de la lentille sur le faisceau de rayons considéré : si elle est positive, la lentille a un effet convergent sur le faisceau de rayons ; si elle est négative, l'effet sur le faisceau de rayons est divergent. Le point de la lentille où l'effet loupe est nul (c'est-à-dire, dans le cas d'une lentille ayant une puissance optique exclusivement sphérique, le point où le rayon incident et le rayon transmis ont même axe) est appelé centre optique.

On définit par ailleurs la « puissance de réfringence cylindrique » d'une lentille pour un rayon incident traversant cette lentille (également appelée puissance optique cylindrique) comme la grandeur qui caractérise et quantifie l'effet de réfringence cylindrique exercé par la lentille sur le rayon considéré, selon lequel il se forme non pas une seule mais deux aires focales, situées dans des plans différents, généralement perpendiculaires entre elles et appelées focales tangentielle et focale sagittale. Cette puissance cylindrique, également appelée « puissance d'astigmatisme » ou simplement « astigmatisme », correspond à la différence des puissances sphériques suivant les deux aires focales. Les deux aires sont repérées par un axe passant par leur « centre optique » communément appelé axe de cylindre.

On définit enfin la « puissance de réfringence prismatique » d'une lentille pour un rayon incident traversant cette lentille (également appelée puissance optique prismatique) comme la grandeur qui caractérise et quantifie l'effet de réfringence prismatique, ou plus simplement de déviation, exercé par la lentille sur le rayon considéré. Cette puissance prismatique, également appelée « prisme », correspond à l'angle de déviation du rayon, c'est-à-dire l'angle formé entre les parties entrantes et sortantes du rayon. Le prisme se décompose en deux composantes : l'une horizontale, appelée prisme horizontal, correspondant à l'angle formé entre les projections des parties entrantes et sortantes du rayon dans le plan horizontal, l'autre verticale, appelée prisme vertical, correspondant à l'angle formé entre les projections des parties entrantes et sortantes du rayon dans le plan vertical.

Dans la suite de cet exposé, le terme « porteur » s'appliquera à toute personne susceptible de porter la monture de lunettes 10. Au moment où est mis en oeuvre le procédé de contrôle selon l'invention, cette personne pourra ne pas encore être identifiée.

On définit les « demi-écarts pupillaires PD » du porteur comme les distances entre l'arête du nez du porteur et chacune de ses pupilles.

On définit également les « hauteurs pupillaires HD » du porteur portant la monture de lunettes 10 comme les distances séparant verticalement ses pupilles d'un trait d'horizon passant par les points les plus bas des deux entourages de la monture de lunettes 10.

Le procédé de contrôle de la faisabilité du montage d'une lentille ophtalmique sur la monture de lunettes 10 comporte les trois étapes suivantes :
a) acquérir une première gamme de valeurs d'au moins un premier paramètre relatif au porteur, dans l'ensemble de laquelle on souhaite assurer la faisabilité de la paire de lunettes,
b) acquérir une seconde gamme de valeurs d'au moins un second paramètre qui est relatif au porteur et qui est distinct de chaque premier paramètre, dans l'ensemble de laquelle on souhaite savoir si la paire de lunettes est faisable,
c) vérifier, pour un nombre caractéristique de valeurs de la seconde gamme, que la paire de lunettes est faisable quelle que soit la valeur de chaque premier paramètre comprise dans sa gamme.

A titre illustratif, un exemple de mise en oeuvre de ce procédé (non compris dans l'invention) pourrait être le suivant :
a) acquérir les bornes minimum et maximum d'une plage de puissance de réfringence sphérique, dans l'ensemble de laquelle on souhaite que la paire de lunettes soit faisable (quelles que soient les valeurs des autres paramètres),
b) acquérir les bornes minimum et maximum d'une plage de puissance de réfringence cylindrique, et
c) vérifier, pour un nombre caractéristique de valeurs de puissance de réfringence cylindrique comprises entre les bornes minimum et maximum, si la paire de lunettes est faisable quelle que soit la valeur de la puissance de réfringence sphérique (pour autant que cette dernière reste comprise entre ses bornes minimum et maximum).

De cette manière, pour savoir si une paire de lunettes est faisable, l'utilisateur n'aura qu'à vérifier que les puissances de réfringence sphérique et cylindriques sont comprises dans les plages identifiées.

Toutefois, le nombre de paramètres à prendre en compte pour que le procédé présente des résultats fiables doit avantageusement être accru.

Ainsi, préférentiellement :
- à l'étape a), on considère deux premiers paramètres dont les valeurs sont comprises dans une première gamme,
- à l'étape b), on considère deux seconds paramètres dont les valeurs sont comprises dans une seconde gamme, et,
- à l'étape c), on vérifie, pour un nombre caractéristique de couples de valeurs des seconds paramètres, que la paire de lunettes est faisable quelles que soient les valeurs des deux premiers paramètres.

On précisera ici qu'une gamme de valeurs peut être formée par un unique point (c'est-à-dire par un unique doublet de valeurs), par un ensemble de points, ou par une infinité de points compris entre des points extrêmes.

En variante, il serait possible de considérer davantage de paramètres.

Ici, les premier et second paramètres sont des paramètres ophtalmiques, morphologiques ou géométrico-morphologiques caractérisant le porteur de lunettes.

Un paramètre ophtalmique est un paramètre relatif aux caractéristiques de vision de l'un des yeux du porteur de lunettes. Typiquement, ce paramètre ophtalmique peut s'exprimer sous la forme d'une puissance de réfringence qu'il faut prévoir sur une lentille ophtalmique pour corriger un défaut de vision du porteur.

Un paramètre morphologique est un paramètre relatif à la forme du visage du porteur de lunettes. Typiquement, un demi-écart pupillaire constitue un tel paramètre morphologique.

Un paramètre géométrico-morphologique est un paramètre qui dépend non seulement de la forme du visage du porteur de lunettes mais aussi de la forme de la monture de lunettes 10. Typiquement, une hauteur pupillaire constitue un tel paramètre géométrico-morphologique.

Dans les modes de réalisation décrits dans la suite de cet exposé, les paramètres pris en compte seront :
- la puissance de réfringence sphérique Ps,
- la puissance de réfringence cylindrique Pc,
- la hauteur pupillaire HD,
- le demi-écart pupillaire PD.

En effet, on estime ici que le caractère « assemblable » d'une paire de lunettes résulte d'un compromis entre les valeurs de ces quatre paramètres, compte tenu de la forme de la monture de lunettes 10 identifiée.

On comprend en effet que les valeurs de puissances de réfringence sphérique Ps et cylindrique Pc ont une influence sur la forme de la lentille ophtalmique et donc sur le caractère faisable d'une paire de lunettes. A titre d'exemple, une lentille ophtalmique très épaisse au niveau de son bord périphérique, ou au contraire très fine, ne pourra ainsi pas être montée dans n'importe quelle monture de lunettes. Une lentille ophtalmique très courbée ne pourra par ailleurs pas être montée dans une monture de lunettes plate, et vis-versa.

Les valeurs de hauteur pupillaire HD et de demi-écart pupillaire PD ont par ailleurs une influence sur la position de la lentille ophtalmique dans l'entourage de la monture de lunettes 10 et donc sur le caractère faisable d'une paire de lunettes. En effet, on comprend qu'une lentille ophtalmique bien centrée dans l'entourage présentera une épaisseur à ses bords qui variera peu, alors qu'une lentille très décentrée présentera une largeur qui variera sensiblement, avec le risque que cette épaisseur soit localement trop faible ou trop importante pour permettre le montage de la lentille ophtalmique dans la monture de lunettes.

En variante, on pourrait également considérer d'autres paramètres supplémentaires, tels que la puissance de réfringence prismatique, puisqu'elle a également une influence sur l'épaisseur de la lentille ophtalmique le long de son bord périphérique.

Pour mieux comprendre l'invention, on peut maintenant décrire plus en détail un premier mode de réalisation du procédé de contrôle, en référence aux figures 1 et 2.

Au cours de l'étape a) (noté Sa sur la figure 1), le calculateur acquiert la première gamme de valeurs, notée Z0.

Cette première gamme Z0, bien illustrée sur la partie gauche de la figure 2, correspond à une plage de doublets de valeurs dans laquelle on souhaite que la paire de lunettes soit faisable.

Ici, les premiers paramètres sont la puissance de réfringence cylindrique Pc et la puissance de réfringence sphérique Ps. Chaque doublet comporte alors en abscisse une valeur de puissance cylindrique Pc et en ordonnée une valeur de puissance de réfringence sphérique Ps. Chaque doublet pourra alors dans la suite de cet exposé être désigné par le terme « point » en ce sens qu'il présente une abscisse et une ordonnée.

Dans ce premier mode de réalisation du procédé de contrôle, on souhaite pouvoir monter dans la monture de lunettes 10 n'importe quelle lentille ophtalmique présentant une puissance de réfringence sphérique Ps et une puissance de réfringence cylindrique Pc dont les valeurs sont comprises dans la première gamme Z0.

Sur la figure 2, on observe que la première gamme Z0 est comprise entre quatre points extrêmes P1, P2, P3, P4.

Ici, à titre illustratif, ces quatre points extrêmes P1, P2, P3, P4 sont définis de la manière suivante :
- au point P1, Pc = 0 δ et Ps = -4 δ (ou « dioptries »),
- au point P2, Pc = 5, 75 δ et Ps = -4 δ,
- au point P3, Pc = 5, 75 δ et Ps = -1,75 δ,
- au point P4, Pc = 0 δ et Ps = 4 δ.

Cette première gamme Z0 comprend alors tous les doublets (Pc, Ps) situés dans le quadrilatère dont les coins correspondent aux quatre points extrêmes P1, P2, P3, P4.

La première gamme Z0 est ici discrétisée par intervalles de 0,25 δ.

Au cours de l'étape b) (noté Sb sur la figure 1), le calculateur acquiert la seconde gamme Z1.

Cette seconde gamme Z1, bien illustrée sur la partie droite de la figure 2, correspond à une plage de doublets de valeurs dans laquelle on souhaite vérifier si la paire de lunettes est ou non faisable.

Ici, les seconds paramètres sont la hauteur pupillaire HD et le demi-écart pupillaire PD. Chaque doublet comporte alors en abscisse une valeur de demi-écart pupillaire PD et en ordonnée une valeur de hauteur pupillaire HD.

Dans ce premier mode de réalisation du procédé de contrôle, on souhaite pouvoir déterminer, dans cette seconde gamme Z1, quelles sont les doublets de valeurs (PD, HD) pour lesquelles il sera possible de monter une lentille ophtalmique dans la monture de lunettes 10, quelles que soient les valeurs de puissances de réfringence sphérique Ps et cylindrique Pc de cette lentille comprises dans la première gamme Z0.

Sur la figure 2, on observe que la seconde gamme Z1 est comprise entre quatre points extrêmes P5, P6, P7, P8.

Ici à titre illustratif, ces quatre points extrêmes P5, P6, P7, P8 sont définis de la manière suivante :
- au point P5, HD = 15 mm et PD = 25 mm,
- au point P6, HD = 15 mm et PD = 37 mm,
- au point P7, HD = 27 mm et PD = 37 mm,
- au point P8, HD = 27 mm et PD = 25 mm.

Cette seconde gamme Z1 comprend alors tous les doublets (PD, HD) situés dans le carré dont les coins correspondent aux quatre points extrêmes P5, P6, P7, P8.

La seconde gamme Z1 est ici discrétisée par intervalles de 1 mm. Autrement formulé, on ne considérera dans la seconde gamme Z1 qu'un nombre limité de points discrétisés.

L'étape c) est quant à elle réalisée en plusieurs opérations successives et itératives, notées Sc1 à Sc8 sur la figure 1.

Cette étape c) consiste à évaluer, pour chaque point discrétisé de la seconde gamme Z1, un niveau de faisabilité Nf de la paire de lunettes.

Ce niveau de faisabilité pourra prendre au moins deux valeurs, par exemple 0 ou 1 selon que la paire de lunettes est ou non faisable.

Ici, ce niveau de faisabilité Nf pourra prendre trois valeurs distinctes, selon que la paire de lunettes est faisable, n'est pas faisable ou est faisable sous condition d'accepter des imperfections de montage.

En variante, ce niveau de faisabilité pourrait prendre davantage de valeurs.

La première opération Sc1 de l'étape c) consiste à déterminer le point Pᵢ à examiner, parmi les points discrétisés de la seconde gamme Z1. Ce point Pᵢ comporte des coordonnées notées (PDᵢ, HDᵢ).

Initialement (pour i=0), les coordonnées (PD₀, HD₀) du point P₀ sont confondues avec celles du point extrême P5.

La seconde opération Sc2 consiste à déterminer si, pour ce point Pᵢ à examiner, le montage d'une lentille ophtalmique (dont les puissances de réfringence sphérique Ps et cylindrique Pc sont comprises dans la première gamme Z0) dans la monture de lunettes 10 est possible.

En pratique, cette seconde opération Sc2 est réalisée en calculant si ce montage est possible lorsque les puissances de réfringence sphérique Ps et cylindrique Pc de la lentille sont égales aux coordonnées du point extrême P1, puis à celles du point extrême P2, puis à celles du point extrême P3, et enfin à celles du point extrême P4.

En effet, on considère que si ces quatre conditions sont réunies, la paire de lunettes sera faisable quelles que soient les valeurs de puissances de réfringence sphérique Ps et cylindrique Pc comprises dans la première gamme Z0.

Chacun de ces quatre calculs est réalisé au moyen d'une fonction mathématique f, qui dépend :
- de la forme de l'entourage 11 de la monture de lunettes,
- des valeurs des puissances de réfringence sphérique Ps et cylindrique Pc au point extrême considéré,
- des valeurs de hauteur pupillaire HD et de demi-écart pupillaire PD au point Pᵢ à examiner, et
- du type de matériau utilisé pour la fabrication des lentilles.

A titre d'exemple illustratif, cette fonction f peut être une fonction polynomiale dont les termes sont constitués par les quatre variables précitées, et dont les coefficients sont ajustés en fonction des tolérances souhaitées.

Plus précisément, les valeurs des coefficients pourront être fixées par défaut égaux à 1, et pourront être ensuite ajustées si nécessaire, en fonction des exigences du fabricant de lentilles et/ou de l'opticien. Ainsi, ces valeurs pourront être différentes selon le niveau de gamme de la paire de lunettes : en effet, une paire de lunettes haut-de-gamme ne devra souffrir d'aucun défaut alors qu'une paire de lunettes milieu-de-gamme pourra présenter des défauts mineurs.

En pratique, cette fonction f permettra toujours de réaliser un tri entre différents montages de paires de lunettes, et l'opticien pourra, parmi ces montages, choisir ceux qu'il considère comme acceptable ou non.

Ici, le résultat de cette fonction f permet d'obtenir le niveau de faisabilité Nf de la paire de lunettes au point Pᵢ à examiner.

Le calculateur pourra ainsi affecter au niveau de faisabilité Nf la valeur 0, 1 ou 2 en fonction du résultat de la fonction f.

Ce niveau de faisabilité Nf peut ainsi être égal à zéro (ce qui est illustré par une croix sur la partie droite de la figure 2), ce qui signifie alors qu'il n'est a priori pas possible (ou pas souhaitable) d'assembler une paire de lunettes pour un porteur ayant une hauteur pupillaire HD et un demi-écart pupillaire dont les valeurs correspondent aux coordonnées du point Pᵢ à examiner, quelles que soient les valeurs des puissances de réfringence sphérique Ps et cylindrique Pc comprises dans la première gamme Z0.

Le niveau de faisabilité Nf peut être égal à un (ce qui est illustré par un point d'interrogation sur la partie droite de la figure 2), ce qui signifie alors qu'il est a priori possible d'assembler une telle paire de lunettes, à condition que le porteur et/ou l'opticien soit prêt à accepter un assemblage imparfait (en termes esthétique ou de solidité d'assemblage).

Enfin, le niveau de faisabilité peut être égal à deux (ce qui est illustré par des cases cochées sur la partie droite de la figure 2), ce qui signifie alors qu'il est a priori possible d'assembler une telle paire de lunettes, en assurant un assemblage parfait.

La troisième opération Sc3 consiste à contrôler si le niveau de faisabilité Nf est égal à 2.

Si tel n'est pas le cas, le calculateur met en oeuvre une opération Sc4 qui consiste à contrôler si le demi-écart pupillaire PDᵢ du point Pᵢ à examiner est égal au demi-écart pupillaire maximum PDₘₐₓ de la seconde gamme Z1.

Si le demi-écart pupillaire PDᵢ n'est pas égal au demi-écart pupillaire maximum PDₘₐₓ, le calculateur met en oeuvre une opération Sc5 qui consiste à incrémenter l'indice i ainsi que la valeur du demi-écart pupillaire PDᵢ₊₁ du point Pᵢ₊₁ à examiner (ici de 1 mm). Les étapes Sc1 et suivantes peuvent alors être répétées, en considérant à l'étape Sc1 le nouveau point Pᵢ₊₁ à examiner.

En revanche, si le demi-écart pupillaire PDᵢ est égal au demi-écart pupillaire maximum PDₘₐₓ, le calculateur met en oeuvre une opération Sc7 décrite ci-après.

A l'issue de l'opération Sc3, si le niveau de faisabilité Nf est égal à 2, le calculateur met en oeuvre une opération Sc6 qui consiste à affecter la valeur 2 au niveau de faisabilité Nf de chacun des points de la seconde gamme Z1 dont :
- l'ordonnée HD est égale à l'ordonnée HDᵢ du point Pᵢ à examiner, et dont
- l'abscisse PD est supérieure à l'abscisse PDᵢ du point Pᵢ à examiner.

A la suite de cette opération Sc6, le calculateur met en oeuvre l'opération Sc7.

Cette opération Sc7 consiste à vérifier si la hauteur pupillaire HDᵢ du point Pᵢ à examiner est égale à la hauteur pupillaire maximum HDₘₐₓ de la seconde gamme Z1.

Si tel est le cas, le procédé s'achève.

En revanche, si tel n'est pas le cas, le calculateur met en oeuvre une opération Sc8 qui consiste à incrémenter l'indice i, à incrémenter la valeur de la hauteur pupillaire HDᵢ₊₁ du point Pᵢ₊₁ à examiner (ici de 1 mm), et à ramener la valeur du demi-écart pupillaire PDᵢ₊₁ à la valeur du demi-écart pupillaire minimum PDₘᵢₙ (ici de 25 mm). Les étapes Sc1 et suivantes peuvent alors être répétées, en considérant à l'étape Sc1 le nouveau point Pᵢ₊₁ à examiner.

A l'issue de cet algorithme, la seconde gamme Z1 se découpe en trois zones distinctes, dont une première zone Z3 dans lequel le montage de la paire de lunettes n'est pas possible, une seconde zone Z2 dans laquelle le montage de la paire de lunettes est possible sous réserve que l'utilisateur soit prêt à accepter que l'assemblage ne soit pas parfait, et une troisième zone Z4 dans laquelle le montage de la paire de lunettes est possible.

De cette manière, lorsqu'un nouveau porteur se présentera et souhaitera acheter une paire de lunettes sur la base de la monture de lunettes 10, il sera possible de lui confirmer très rapidement si sa paire de lunettes pourra être assemblée, en vérifiant simplement que ses prescriptions (en termes de puissance de réfringence sphérique Ps et de puissance de réfringence cylindrique Pc) sont comprises dans la première gamme Z0 et que ses hauteur pupillaire HD et demi-écart pupillaires PD sont comprises dans la zone Z4 de la seconde gamme Z1.

Si ses hauteur pupillaire HD et demi-écart pupillaires PD sont comprises dans la zone Z2 de la seconde gamme Z1, l'utilisateur pourra proposer au nouveau porteur soit de choisir une autre monture de lunettes, soit de conserver ce choix de monture en acceptant toutefois que sa paire de lunettes assemblée présente quelques imperfections de montage.

On pourra enfin noter que les valeurs des points extrêmes P1, P2, P3, P4 délimitant la première gamme Z0 pourront être déterminées soit de manière aléatoire, en fonction de la volonté de l'utilisateur, soit automatiquement, en fonction de la forme de la monture de lunettes 10.

On comprend en effet que selon la forme (et notamment de la courbure) de la monture de lunettes 10, il ne sera pas esthétique ou possible d'y monter n'importe quelle lentille ophtalmique.

Alors, au cours d'une étape préalable à l'étape a), le calculateur pourra mettre en oeuvre une étape d'acquisition d'une valeur caractéristique de la courbure générale de la monture de lunettes 10, puis, à l'étape a), il pourra déterminer les valeurs des coordonnées des points extrêmes P1, P2, P3, P4 en fonction de cette valeur caractéristique.

A titre d'exemple non limitatif, lorsque la monture de lunettes est particulièrement courbée, il sera possible de limiter la plage Z0 à des valeurs de puissances sphériques positives.

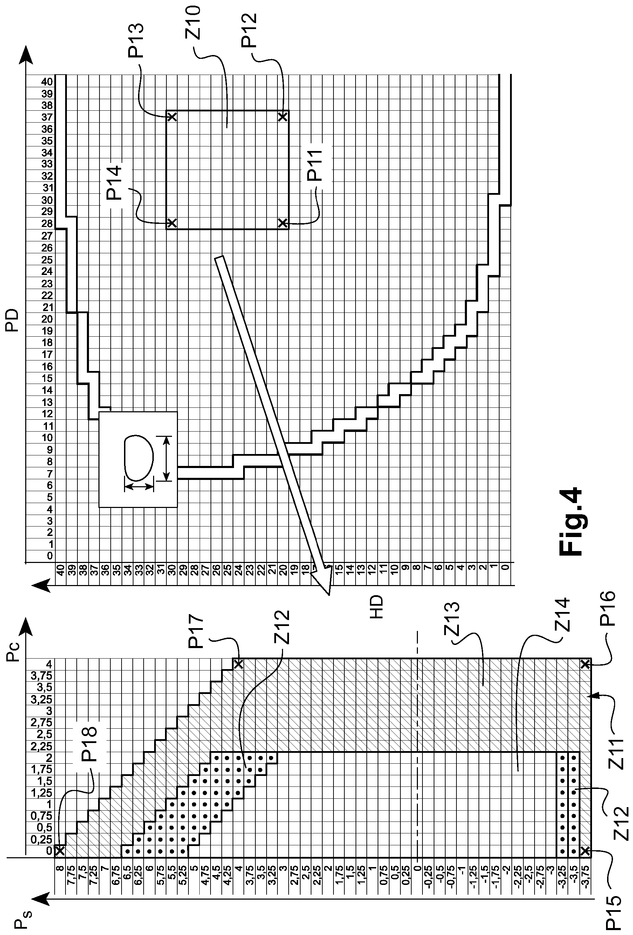

On peut maintenant décrire un second mode de réalisation du procédé de contrôle conforme à l'invention, en référence aux figures 3 et 4.

Au cours de l'étape a) (noté Sa sur la figure 3), le calculateur acquiert la première gamme de valeurs, notée Z10.

Cette première gamme Z10, bien illustrée sur la partie droite de la figure 4, correspond à une plage de doublets de valeurs dans laquelle on souhaite que la paire de lunettes soit faisable.

Ici, les premiers paramètres sont la hauteur pupillaire HD et le demi-écart pupillaire PD. Chaque doublet comporte alors en abscisse une valeur de demi-écart pupillaire PD et en ordonnée une valeur de hauteur pupillaire HD.

Dans ce second mode de réalisation du procédé de contrôle, on souhaite que les valeurs des hauteur pupillaire HD et demi-écart pupillaire PD ne soient pas discriminantes au moment du choix de la monture de lunettes 10 par un client (pour autant que ces valeurs restent comprises dans la première gamme Z10).

Sur la figure 2, on observe que la première gamme Z10 est comprise entre quatre points extrêmes P11, P12, P13, P14.

Ici, à titre illustratif, ces quatre points extrêmes P11, P12, P13, P14 sont définis de la manière suivante :
- au point P11, HD = 20 mm et PD = 28 mm,
- au point P12, HD = 20 mm et PD = 37 mm,
- au point P13, HD = 30 mm et PD = 37 mm,
- au point P14, HD = 30 mm et PD = 28 mm.

Cette première gamme Z10 comprend alors tous les doublets (HD, PD) situés dans le carré dont les coins correspondent aux quatre points extrêmes P11, P12, P13, P14.

Ici, la première gamme Z10 est ici discrétisée par intervalles de 1 mm.

Au cours de l'étape b) (noté Sb sur la figure 3), le calculateur acquiert la seconde gamme Z11.

Cette seconde gamme Z11, bien illustrée sur la partie gauche de la figure 4, correspond à une plage de doublets de valeurs dans laquelle on souhaite vérifier si la paire de lunettes est ou non faisable.

Ici, les seconds paramètres sont la puissance de réfringence cylindrique Pc et la puissance de réfringence sphérique Ps. Chaque doublet comporte alors en abscisse une valeur de puissance cylindrique Pc et en ordonnée une valeur de puissance de réfringence sphérique Ps.

Dans ce second mode de réalisation du procédé de contrôle, on souhaite en effet pouvoir déterminer, dans cette seconde gamme Z11, quelles sont les doublets de valeurs (Ps, Pc) pour lesquelles il sera possible de monter une lentille ophtalmique dans la monture de lunettes 10, quelles que soient les valeurs des doublets (HD, PD) compris dans la première gamme Z10.

Sur la figure 4, on observe que la seconde gamme Z11 est comprise entre quatre points extrêmes P15, P16, P17, P18.

Ici à titre illustratif, ces quatre points extrêmes P15, P16, P17, P18 sont définis de la manière suivante :
- au point P15, Pc = 0 δ et Ps = -3,75 δ,
- au point P16, Pc = 4 δ et Ps = -3,75 δ,
- au point P17, Pc = 4 δ et Ps = 4 δ,
- au point P18, Pc = 0 δ et Ps = 8 δ.

Cette seconde gamme Z11 comprend alors tous les doublets (Ps, Pc) situés dans le quadrilatère dont les coins correspondent aux quatre points extrêmes P15, P16, P17, P18.

La seconde gamme Z11 est ici discrétisée par intervalles de 0,25 δ.

L'étape c) est quant à elle réalisée en plusieurs opérations successives et itératives.

Cette étape consiste à évaluer, pour chaque point discrétisé de la seconde gamme Z11, un niveau de faisabilité Nf de la paire de lunettes.

Ce niveau de faisabilité prendra ici encore trois valeurs distinctes, selon que la paire de lunettes est faisable, n'est pas faisable ou est faisable sous condition d'accepter des imperfections de montage.

Au cours de cette étape c), le calculateur va parcourir l'ensemble des points discrétisés de la seconde gamme Z11, en calculant à chaque fois la valeur de ce niveau de faisabilité Nf.

Pour cela, contrairement au procédé mis en oeuvre dans le premier mode de réalisation, ici, cette étape c) est réalisée en deux sous-étapes, selon que la valeur de puissance sphérique Ps des points Pᵢ à examiner est positive ou négative.

On pourra ici considérer uniquement le cas des points Pᵢ à examiner pour lesquelles la puissance sphérique Ps est positive (partie encadrée de l'organigramme de la figure 3). La mis en oeuvre du procédé pour les autres points à examiner se fera en effet de manière homologue.

La première opération Sc1 de l'étape c) consiste à déterminer le point Pᵢ à examiner, parmi les points discrétisés de la seconde gamme Z11. Ce point Pᵢ comporte des coordonnées notées (Pcᵢ, Psᵢ).

Initialement (pour i=0), les coordonnées (Pc₀, Ps₀) du point P₀ sont confondues avec celles du point extrême P18.

La seconde opération Sc2 consiste à déterminer si, pour ce point Pᵢ à examiner, le montage d'une lentille ophtalmique dans la monture de lunettes 10 est possible quelles que soient les hauteur pupillaire HD et demi-écart pupillaire PD compris dans la première gamme Z10.

En pratique, cette seconde opération Sc2 est réalisée en calculant si ce montage est possible lorsque hauteur pupillaire HD et demi-écart pupillaire PD sont égales aux coordonnées du point extrême P11, puis à celles du point extrême P12, puis à celles du point extrême P13, et enfin à celles du point extrême P14.

Chacun de ces quatre calculs est réalisé au moyen d'une fonction mathématique f, qui dépend :
- de la forme de l'entourage 11 de la monture de lunettes,
- des valeurs des puissances de réfringence sphérique Ps et cylindrique Pc au point Pᵢ à examiner,
- des valeurs des hauteur pupillaire HD et demi-écart pupillaire PD au point extrême considéré, et
- du type de matériau utilisé pour la fabrication des lentilles.

Le résultat de cette fonction f permet d'obtenir le niveau de faisabilité Nf de la paire de lunettes au point Pᵢ à examiner.

Ce niveau de faisabilité Nf peut être égal à zéro, ce qui signifie alors qu'il n'est a priori pas possible d'assembler à la monture de lunettes 10 une lentille ophtalmique ayant des puissances de réfringence sphérique Ps et cylindrique Pc dont les valeurs correspondent aux coordonnées du point Pᵢ à examiner, quelles que soient les valeurs des hauteur pupillaire HD et demi-écart pupillaire PD comprises dans la première gamme Z10.

Le niveau de faisabilité Nf peut être égal à un, ce qui signifie alors qu'il est a priori possible d'assembler une telle paire de lunettes, à condition que l'utilisateur soit prêt à accepter un assemblage imparfait (en termes esthétique ou de solidité d'assemblage).

Enfin, le niveau de faisabilité peut être égal à deux, ce qui signifie alors qu'il est a priori possible d'assembler une telle paire de lunettes, en assurant un assemblage parfait.

La troisième opération Sc3 consiste à contrôler si les coordonnées du point Pᵢ à examiner sont égales à (4δ, 0 δ).

Si tel n'est pas le cas, le calculateur met en oeuvre une opération Sc4 qui consiste à incrémenter l'indice i. Les étapes Sc1 et suivantes peuvent alors être répétées, en considérant à l'étape Sc1 un nouveau point Pᵢ₊₁ à examiner.

La définition de ce nouveau point Pᵢ₊₁ consiste à :
- incrémenter de 0,25 δ la puissance cylindrique Pc tant que le côté du quadrilatère n'a pas été atteint, ou à
- décrémenter de 0,25 δ la puissance sphérique Ps et à ramener la puissance cylindrique Pc à 0 δ si le côté du quadrilatère a été atteint.

A l'issue de l'opération Sc3, si les coordonnées du point Pᵢ à examiner sont égales à (4δ, 0 δ), le calculateur met en oeuvre une opération Sc5 d'attente que tous les points Pi aient été examinées et il déclenche une temporisation.

L'examen des points pour lesquels la puissance sphérique Ps est négative est en effet opéré simultanément à l'examen des points pour lesquels la puissance sphérique Ps est positive ; il est alors nécessaire d'attendre que tous les calculs s'achèvent.

Si l'ensemble des calculs est effectué avant la fin de la temporisation, le calculateur stocke dans sa mémoire vive l'ensemble des résultats (opérations Sc6).

Si l'ensemble des calculs n'est pas effectué avant la fin de la temporisation (ce qui indique qu'il y a un problème), le calculateur interrompt le procédé (opérations Sc7).

On notera ici que pour réduire le nombre de calculs à effectuer, le calculateur pourra, lorsqu'un point Pi à examiner présente un niveau de faisabilité Nf égal à zéro, affecter la valeur 0 au niveau de faisabilité Nf de chacun des points de la seconde gamme Z11 dont :
- l'ordonnée Ps est égale à l'ordonnée Psᵢ du point Pᵢ à examiner, et dont
- l'abscisse Pc est supérieure à l'abscisse Pcᵢ du point Pᵢ à examiner.

Dans le même but, le calculateur pourra également, lorsqu'un point Pᵢ à examiner présente un niveau de faisabilité Nf égal à zéro, affecter la valeur 0 au niveau de faisabilité Nf de chacun des points Pₖ de la seconde gamme Z11 dont :
- l'abscisse Pcₖ est égale à la somme d'un multiple du pas (0,25 δ) et de l'abscisse Pcᵢ du point Pᵢ à examiner, et dont
- l'ordonnée Psₖ est égale à l'ordonnée Psᵢ du point Pᵢ à examiner, retranchée du même multiple du pas (0,25 δ),
ce que l'on peut écrire :
Nf (Pₖ) = 0 si Psₖ = Psᵢ - k . 0,25 et si Pcₖ = Pcᵢ + k . 0,25

On pourra enfin noter que les valeurs des points extrêmes P11, P12, P13, P14 délimitant la première gamme Z10 pourront être déterminées soit de manière aléatoire, en fonction de la volonté de l'utilisateur, soit automatiquement, en fonction de données géomorphologiques relatives à une région donnée du globe terrestre.

On observe en effet que les demi-écarts pupillaires et les hauteurs pupillaires moyennes varient d'une région du globe à l'autre.

Alors, au cours d'une étape préalable à l'étape a), le calculateur pourra mettre en oeuvre une étape d'acquisition des valeurs moyennes des demi-écarts pupillaires et des hauteurs pupillaires dans la région où la vente de la monture de lunettes aura lieu, puis, à l'étape a), il pourra déterminer les valeurs des coordonnées des points extrêmes P11, P12, P13, P14 en fonction de ces valeurs moyennes.

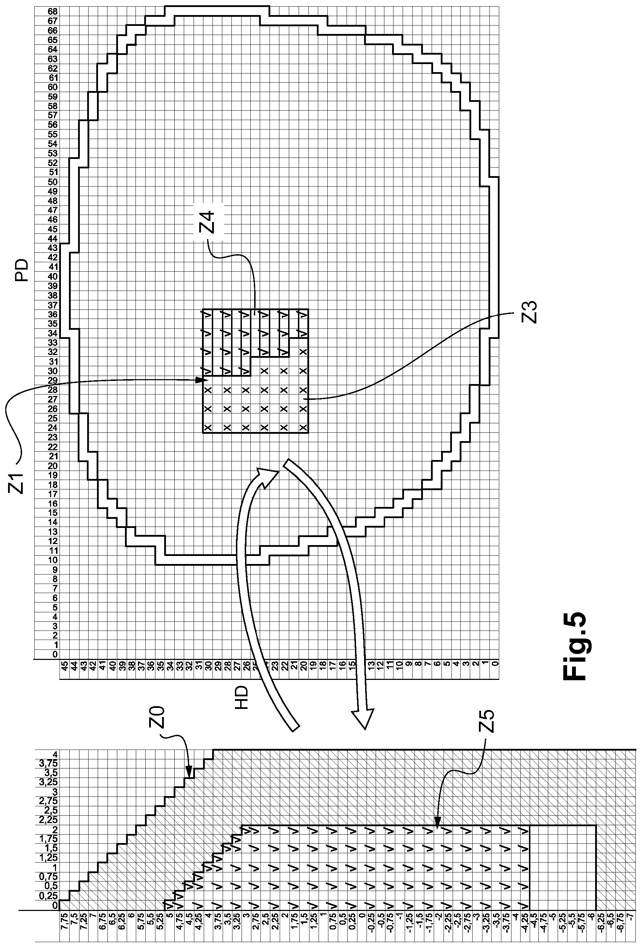

On pourra également décrire un troisième mode de réalisation du procédé de contrôle conforme à l'invention, en référence à la figure 5.

Dans ce troisième mode de réalisation, le but va consister, lorsque le niveau de faisabilité Nf de la paire de lunettes est égal à zéro au niveau d'un point Pᵢ à examiner, à vérifier s'il ne pourrait pas être égal à 2 si on réduisait la première gamme. On va donc chercher une sous-gamme de la première gamme pour laquelle l'ensemble des niveaux de faisabilité Nf des points de la seconde gamme seront égaux à 2.

La mise en oeuvre de ce troisième mode de réalisation du procédé de contrôle se fera de la manière suivante.

La première opération réalisée par le calculateur consiste à mettre en oeuvre le premier mode de réalisation du procédé décrit supra, en considérant toutefois que le niveau de faisabilité Nf de la paire de lunettes est soit égal à zéro (si la paire de lunettes n'est pas faisable), soit égal à deux (si la paire de lunettes est faisable).

Comme le montre la figure 5, la seconde gamme Z1 comprend alors une zone Z4 dans laquelle la paire de lunettes est faisable, et une seconde zone Z3 dans laquelle la paire de lunettes n'est pas faisable.

Pour les points de cette seconde zone Z3, on va alors chercher à déterminer parmi les points discrétisés de la première gamme Z0, ceux pour lesquels l'assemblage de la paire de lunettes serait faisable.

En effet, à l'issus du premier mode de réalisation du procédé de contrôle, on sait que si les hauteur pupillaire HD et demi-écart pupillaire PD du porteur sont comprises dans la zone Z4 de la seconde gamme Z1 et que les puissances de réfringence sphérique Ps et cylindrique Pc sont comprises dans la première gamme Z0, il sera possible d'assembler la paire de lunettes sans défaut.

Cette fois, le procédé va être complété par une quatrième étape d) permettant de déterminer, dans la première gamme Z0, les points pour lesquelles la paire de lunettes sera faisable lorsque les hauteur pupillaire HD et demi-écart pupillaire PD du porteur seront compris dans la zone Z3 de la seconde gamme Z1.

En pratique, cette étape d) va consister, pour le calculateur, à mettre en oeuvre le second mode de réalisation du procédé de contrôle illustré sur les figures 3 et 4.

Il sera ainsi possible de déterminer, dans la première gamme Z0, une zone Z5 (ou « sous-gamme ») dans l'ensemble de laquelle la paire de lunettes sera faisable, quelles que soient les valeurs de hauteur pupillaire HD et demi-écart pupillaire PD comprises dans la seconde gamme Z1.

En variante, il aurait été possible d'inverser les étapes, en mettant tout d'abord en oeuvre le second mode de réalisation du procédé de contrôle illustré sur les figures 3 et 4, puis en mettant en oeuvre, au cours d'une étape d), le premier mode de réalisation du procédé de contrôle illustré sur les figures 1 et 2.

On pourra enfin décrire un quatrième mode de réalisation du procédé de contrôle conforme à l'invention, non illustré sur les figures.

Dans ce quatrième mode de réalisation, le but va consister, lorsque le niveau de faisabilité Nf de la paire de lunettes est égal à 2 au niveau d'un point Pᵢ à examiner, à vérifier s'il ne pourrait pas aussi être égal à 2 si on élargissait la première gamme. On va donc chercher une « sur-gamme » englobant la première gamme dans laquelle l'ensemble des points qui avaient un niveau de faisabilité Nf égal à 2 conserveront ce niveau de faisabilité Nf.

La mise en oeuvre de ce quatrième mode de réalisation du procédé de contrôle se fera de la manière suivante.

La première opération réalisée par le calculateur consiste ici encore à mettre en oeuvre le premier mode de réalisation du procédé décrit supra, en considérant toutefois que le niveau de faisabilité Nf de la paire de lunettes est soit égal à zéro (si la paire de lunettes n'est pas faisable), soit égal à deux (si la paire de lunettes est faisable).

La seconde gamme Z1 comprend alors une zone Z4 dans laquelle la paire de lunettes est faisable, et une seconde zone Z3 dans laquelle la paire de lunettes n'est pas faisable.

Cette fois, le procédé va être complété par une quatrième étape d) permettant de déterminer, dans une sur-gamme englobant la première gamme Z0, les points dans lesquelles la paire de lunettes serait faisable lorsque les hauteur pupillaire HD et demi-écart pupillaire PD du porteur seront comprises dans la zone Z4 de la seconde gamme Z1.

En pratique, cette étape d) va consister, pour le calculateur, à mettre en oeuvre le second mode de réalisation du procédé de contrôle illustré sur les figures 3 et 4.

Il sera ainsi possible de déterminer, dans la sur-gamme englobant la première gamme Z0, une zone dans l'ensemble de laquelle la paire de lunettes sera faisable, quelles que soient les valeurs de hauteur pupillaire HD et de demi-écart pupillaire PD comprises dans la zone Z2 de la seconde gamme Z1.

Quel que soit le mode de réalisation utilisé, le procédé de contrôle permet de déterminer une zone de la première gamme Z0 et une zone de la seconde gamme Z1 dans lesquelles l'assemblage de la paire de lunettes sera possible.

Cette méthode de contrôle pourra alors être répétée avec d'autres montures de lunettes 10, de manière à obtenir un catalogue de montures de lunettes dans lequel il sera facile de déterminer les montures de lunettes qui pourront être proposées à un porteur, compte tenu de ses prescriptions.

C'est à cet effet que pourra être utilisé le registre de base de données mémorisé dans la mémoire morte du calculateur, dont on rappelle qu'il comporte plusieurs enregistrements associant chacun, à une référence de monture de lunettes 10, des première et seconde gammes.

Plus précisément, après avoir mis en oeuvre le procédé de contrôle sur une monture de lunettes 10 d'une nouvelle référence, le calculateur pourra enregistrer (dans un nouvel enregistrement de la base de données associé à ladite nouvelle référence) des données relatives aux zones validées des première et seconde gammes Z0, Z1 (celles dans lesquelles l'assemblage de la paire de lunettes est possible).

On peut maintenant considérer le cas d'un opticien mettant à disposition de ses clients plusieurs montures de lunettes chacune référencée dans le registre de base de données.

Alors, lorsqu'un nouveau porteur se présentera avec ses prescriptions (puissance de réfringence sphérique et cylindrique, hauteur et demi-écart pupillaires), l'opticien pourra saisir ces prescriptions sur les moyens d'acquisition du calculateur, de manière que le calculateur puisse mettre en oeuvre une opération de sélection des enregistrements de la base de données pour lesquels les prescriptions sont inclues dans les zones validées des première et seconde gammes Z0, Z1.

De cette manière, l'opticien pourra communiquer au porteur quelles montures de lunettes il pourra choisir en étant certain d'obtenir une bonne qualité d'assemblage, quelles montures de lunettes il pourra choisir s'il est prêt à accepter une qualité moyenne d'assemblage, et quelles montures de lunettes il ne pourra pas choisir.

Pour indiquer facilement quelles montures de lunettes le porteur pourra choisir, on pourra prévoir que l'opticien mette à disposition du porteur soit un catalogue électronique de montures de lunettes, soit qu'il communique au porteur un indicateur de faisabilité.

Considérons tout d'abord le cas du catalogue électronique.

En pratique, l'opticien mettra à disposition du porteur un ordinateur ayant accès aux enregistrements du registre de base de données pour lesquels les prescriptions sont inclues dans les zones validées des première et seconde gammes Z0, Z1.

Pour rendre ce catalogue agréable à consulter, on pourra prévoir que chaque enregistrement mémorise un identifiant de la monture de lunettes (par exemple le nom du modèle de la monture) et au moins une photo de la monture de lunettes associée à cet enregistrement.

Considérons maintenant le cas de l'indicateur de faisabilité.

On pourra prévoir que l'opticien, à chaque fois qu'il met en rayon une nouvelle monture de lunettes, appose sur celle-ci un indicateur de faisabilité (par exemple sous la forme d'une note comprise entre 1/10 et 10/10, ou une gommette de couleur).

Cet indicateur de faisabilité sera au préalable déterminé en fonction des zones validées des première et seconde gammes Z0, Z1 (par exemple en fonction de leurs amplitudes ou en fonction de leurs valeurs).

Ainsi, après que l'opticien aura saisi les prescriptions du porteur dans le calculateur, ce dernier pourra calculer à quels indicateurs de faisabilité le porteur aura accès.

A titre d'exemple, un porteur ayant besoin d'une puissance sphérique faible et d'une puissance cylindrique nulle pourrait avoir accès à toutes les montures ayant une note comprise entre 1/10 et 10/10, tandis qu'un porteur ayant besoin d'une puissance sphérique forte et d'une puissance cylindrique élevée ne pourrait avoir accès qu'aux montures ayant une note comprise entre 9/10 et 10/10.

## Revendications

1. Procédé de contrôle de la faisabilité d'une paire de lunettes mis en oeuvre par un dispositif électronique et/ou informatique, dans le but de mettre à disposition d'un porteur identifié plusieurs montures de paires de lunettes faisables, cette paire de lunettes comprenant une monture de lunettes (10) physique et identifiée présentant au moins un entourage (11) adaptée à recevoir une lentille, comportant une étape consistant à :
a) acquérir une gamme de valeurs d'au moins un premier paramètre (PD, HD, Ps, Pc) relatif à un quelconque porteur de lunettes, dans l'ensemble de laquelle on souhaite assurer la faisabilité de la paire de lunettes,
**caractérisé en ce qu'**il comporte en outre des étapes consistant à :
b) acquérir une gamme de valeurs d'au moins un second paramètre (PD, HD, Ps, Pc) qui est relatif à un quelconque porteur de lunette et qui est distinct de chaque premier paramètre, dans l'ensemble de laquelle on souhaite s'assurer de la faisabilité de la paire de lunettes,
c) vérifier, en fonction de la forme de l'entourage (11) et pour un nombre caractéristique de valeurs de chaque second paramètre (PD, HD, Ps, Pc), que la paire de lunettes est faisable quelle que soit la valeur de chaque premier paramètre (PD, HD, Ps, Pc) comprise dans sa gamme de manière que, lorsque le porteur identifié souhaite acheter une paire de lunettes basée sur ladite monture de lunettes (10), il soit possible de lui confirmer si ladite paire de lunettes est faisable en déterminant les valeurs desdits premier et second paramètres et en vérifiant que la valeur du premier paramètre est comprise dans ladite gamme acquise à l'étape a) et que la valeur du second paramètre est comprise dans ladite gamme acquise à l'étape b) et est telle que la paire de lunettes est faisable au sens de l'étape c), dans lequel lesdits premier et second paramètres (PD, HD, Ps, Pc) sont des paramètres ophtalmiques, morphologiques ou géométrico-morphologiques caractérisant des porteurs de lunettes,
dans lequel lesdits premiers paramètres incluent la puissance de réfringence sphérique (Ps) et la puissance de réfringence cylindrique (Pc), et
dans lequel ledit ou lesdits seconds paramètres comprennent la hauteur pupillaire (HD) et/ou le demi-écart pupillaire (PD).

2. Procédé de contrôle selon la revendication précédente, dans lequel lesdits seconds paramètres incluent la hauteur pupillaire (HD)et le demi-écart pupillaire (PD).

3. Procédé de contrôle selon l'une des revendications précédentes, dans lequel :
- à l'étape a), on acquiert une gamme de valeurs de deux premiers paramètres (PD, HD, Ps, Pc), et
- à l'étape c), on vérifie, pour un nombre caractéristiques de valeurs de chaque second paramètre (PD, HD, Ps, Pc), que la paire de lunettes est faisable quelles que soient les valeurs des deux premiers paramètres (PD, HD, Ps, Pc) comprises dans leur gamme.

4. Procédé de contrôle selon l'une des revendications précédentes, dans lequel :
- à l'étape b), on acquiert une gamme de valeurs de deux seconds paramètres (PD, HD, Ps, Pc), et
- à l'étape c), on vérifie, pour un nombre caractéristiques de couples de valeurs des seconds paramètres, que la paire de lunettes est faisable quelle que soit la valeur du premier paramètre (PD, HD, Ps, Pc) comprise dans sa gamme.

5. Procédé de contrôle selon l'une des revendications précédentes, dans lequel, à l'étape c), on évalue un niveau de faisabilité de la paire de lunettes, ledit niveau de faisabilité pouvant prendre au moins trois valeurs distinctes.

6. Procédé de contrôle selon l'une des revendications précédentes, dans lequel il est prévu, préalablement à l'étape a), une étape d'acquisition d'une donnée de courbure de la monture de lunettes (10) et dans lequel, à l'étape a), la gamme de valeurs des premiers paramètres est choisie en fonction de ladite donnée de courbure de la monture de lunettes (10).

7. Procédé de contrôle selon l'une des revendications précédentes, dans lequel, considérant une sous-gamme de la gamme de valeurs de chaque second paramètre (PD, HD, Ps, Pc) dans laquelle la paire de lunettes n'est pas faisable, il est prévu une étape consistant à :
d) vérifier, pour un nombre caractéristiques de valeurs de chaque premier paramètre (PD, HD, Ps, Pc), que la paire de lunettes est faisable quelle que soit la valeur de chaque second paramètre (PD, HD, Ps, Pc) compris dans ladite sous-gamme.

8. Procédé de contrôle selon l'une des revendications précédentes, dans lequel, considérant une sous-gamme de la gamme de valeurs de chaque second paramètre (PD, HD, Ps, Pc) dans laquelle la paire de lunettes est faisable et une sur-gamme englobant la gamme de valeurs du premier paramètre (PD, HD, Ps, Pc), il est prévu une étape consistant à :
d) vérifier, pour un nombre caractéristiques de valeurs du premier paramètre (PD, HD, Ps, Pc) compris dans ladite sur-gamme, que la paire de lunettes est faisable quelle que soit la valeur de chaque second paramètre (PD, HD, Ps, Pc) compris dans ladite sous-gamme.

9. Méthode de classement de montures de lunettes (10), au moyen d'une base de données comprenant des enregistrements chacun associés à une monture de lunettes, comprenant :
- une opération de calcul au cours de laquelle on met en oeuvre le procédé de contrôle selon l'une des revendications précédentes pour une monture de lunettes (10) identifiée, et
- une opération de stockage au cours de laquelle on enregistre, dans un nouvel enregistrement de la base de données associé à ladite monture de lunettes (10) identifiée, la gamme de valeurs de chaque premier paramètre ainsi qu'une gamme de valeurs de chaque second paramètre dans l'ensemble de laquelle la paire de lunettes est faisable, dans le but de mettre à disposition d'un porteur identifié plusieurs montures de paires de lunettes faisables.

10. Méthode de classement selon la revendication précédente, dans lequel il est prévu :
- une opération d'acquisition d'une première valeur particulière du premier paramètre et d'une seconde valeur particulière du second paramètre, ces première et seconde valeurs correspondant à un porteur de lunettes donné, et
- une opération de sélection des enregistrements de ladite base de données dans lesquels les gammes mémorisées comprennent lesdites première et seconde valeurs particulières.

## Patentansprüche

1. Verfahren zur Prüfung der Machbarkeit einer Brille, das von einer elektronischen und/oder informationstechnischen Vorrichtung eingesetzt wird, um einem identifizierten Träger mehrere machbare Brillengestelle zur Verfügung zu stellen, wobei diese Brille ein physisches und identifiziertes Brillengestell (10) umfasst, das mindestens eine Fassung (11) aufweist, die zum Aufnehmen einer Linse geeignet ist, umfassend einen Schritt, der darin besteht:
a) einen Wertebereich mindestens eines ersten Parameters (PD, HD, Ps, Pc) bezüglich eines beliebigen Brillenträgers zu erfassen, in dem man die Machbarkeit der Brille gewährleisten möchte,
**dadurch gekennzeichnet, dass** es ferner Schritte umfasst, die darin bestehen:
b) einen Wertebereich mindestens eines zweiten Parameters (PD, HD, Ps, Pc), der sich auf einen beliebigen Brillenträger bezieht und der von dem ersten Parameter verschieden ist, zu erfassen, in dem man sich der Machbarkeit der Brille vergewissern möchte,
c) in Abhängigkeit von der Form der Fassung (11) und für eine charakteristische Anzahl von Werten jedes zweiten Parameters (PD, HD, Ps, Pc) zu überprüfen, dass die Brille machbar ist, unabhängig von dem Wert jedes ersten Parameters (PD, HD, Ps, Pc), der in seinem Bereich enthalten ist, so dass, wenn der identifizierte Träger eine auf dem Brillengestell (10) beruhende Brille kaufen möchte, es möglich ist, ihm zu bestätigen, ob die Brille machbar ist, indem die Werte der ersten und zweiten Parameter bestimmt werden und indem überprüft wird, dass der Wert des ersten Parameters in dem im Schritt a) erfassten Bereich enthalten ist und dass der Wert des zweiten Parameters in dem im Schritt b) erfassten Bereich enthalten ist und dergestalt ist, dass die Brille im Sinne des Schritts c) machbar ist, wobei die ersten und zweiten Parameter (PD, HD, Ps, Pc) ophthalmische, morphologische oder geometrisch-morphologische Parameter sind, die Brillenträger kennzeichnen,
wobei die ersten Parameter die sphärische Brechkraft (Ps) und die zylindrische Brechkraft (Pc) beinhalten, und
wobei die ersten oder die zweiten Parameter die Pupillenhöhe (HD) und/oder den halben Pupillenabstand (PD) umfassen.

2. Verfahren zur Prüfung nach dem vorhergehenden Anspruch, wobei die zweiten Parameter die Pupillenhöhe (HD) und den halben Pupillenabstand (PD) beinhalten.

3. Verfahren zur Prüfung nach einem der vorhergehenden Ansprüche, wobei:
- im Schritt a) ein Wertebereich von zwei ersten Parametern (PD, HD, Ps, Pc) erfasst wird, und
- im Schritt c) für eine charakteristische Anzahl von Werten jedes zweiten Parameters (PD, HD, Ps, Pc) überprüft wird, dass die Brille machbar ist, unabhängig von den Werten der zwei ersten Parameter (PD, HD, Ps, Pc), die in ihrem Bereich enthalten sind.

4. Verfahren zur Prüfung nach einem der vorhergehenden Ansprüche, wobei:
- im Schritt b) ein Wertebereich von zwei zweiten Parametern (PD, HD, Ps, Pc) erfasst wird, und
- im Schritt c) für eine charakteristische Anzahl von Wertepaaren der zweiten Parameter überprüft wird, dass die Brille machbar ist, unabhängig von dem Wert des ersten Parameters (PD, HD, Ps, Pc), der in seinem Bereich enthalten ist.

5. Verfahren zur Prüfung nach einem der vorhergehenden Ansprüche, wobei im Schritt c) ein Machbarkeitsniveau der Brille bewertet wird, wobei das Machbarkeitsniveau mindestens drei verschiedene Werte annehmen kann.

6. Verfahren zur Prüfung nach einem der vorhergehenden Ansprüche, wobei vor dem Schritt a) ein Schritt des Erfassens eines Krümmungsdatenwerts des Brillengestells (10) vorgesehen ist und wobei im Schritt a) der Wertebereich der ersten Parameter in Abhängigkeit von dem Krümmungsdatenwert des Brillengestells (10) ausgewählt wird.

7. Verfahren zur Prüfung nach einem der vorhergehenden Ansprüche, wobei, in Anbetracht eines Unterbereichs des Wertebereichs jedes zweiten Parameters (PD, HD, Ps, Pc), in dem die Brille nicht machbar ist, ein Schritt vorgesehen ist, der darin besteht:
d) für eine charakteristische Anzahl von Werten jedes ersten Parameters (PD, HD, Ps, Pc) zu überprüfen, dass die Brille machbar ist, unabhängig von dem Wert jedes zweiten Parameters (PD, HD, Ps, Pc), der in dem Unterbereich enthalten ist.

8. Verfahren zur Prüfung nach einem der vorhergehenden Ansprüche, wobei, in Anbetracht eines Unterbereichs des Wertebereichs jedes zweiten Parameters (PD, HD, Ps, Pc), in dem die Brille machbar ist, und eines Überbereichs, der den Wertebereich des ersten Parameters (PD, HD, Ps, Pc) einschließt, ein Schritt vorgesehen ist, der darin besteht:
d) für eine charakteristische Anzahl von Werten des ersten Parameters (PD, HD, Ps, Pc), die in dem Überbereich enthalten sind, zu überprüfen, dass die Brille machbar ist, unabhängig von dem Wert jedes zweiten Parameters (PD, HD, Ps, Pc), der in dem Unterbereich enthalten ist.

9. Methode zur Klassifizierung von Brillengestellen (10) mittels einer Datenbank, die Datensätze umfasst, die jeweils einem Brillengestell zugeordnet sind, umfassend:
- einen Rechenvorgang, in dessen Verlauf man das Verfahren zur Prüfung nach einem der vorhergehenden Ansprüche für ein identifiziertes Brillengestell (10) einsetzt, und
- einen Speichervorgang, in dessen Verlauf man in einem neuen Datensatz der Datenbank, der dem identifizierten Brillengestell (10) zugeordnet ist, den Wertebereich jedes ersten Parameters sowie einen Wertebereich jedes zweiten Parameters, in dem die Brille machbar ist, speichert, um einem identifizierten Träger mehrere machbare Brillengestelle zur Verfügung zu stellen.

10. Methode zur Klassifizierung nach dem vorhergehenden Anspruch, wobei vorgesehen ist:
- ein Vorgang des Erfassens eines ersten besonderen Werts des ersten Parameters und eines zweiten besonderen Werts des zweiten Parameters, wobei diese ersten und zweiten Werte einem gegebenen Brillenträger entsprechen, und
- ein Vorgang des Auswählens der Datensätze in der Datenbank, in denen die gespeicherten Bereiche die ersten und zweiten besonderen Werte umfassen.

## Claims

1. Process for testing the feasibility of a pair of spectacles carried out by an electronic and/or computer device, for the purpose of making several feasible pairs of spectacle frames available to an identified wearer, this pair of spectacles comprising an identified physical spectacle frame (10) having at least one eye wire (11) suitable for receiving a lens, comprising a step consisting in:
a) acquiring a range of values of at least one first parameter (PD, HD, Ps, Pc) relating to any spectacle wearer, in the whole of which range it is desired to ensure the feasibility of the pair of spectacles,
**characterized in that** it further comprises steps consisting in:
b) acquiring a range of values of at least one second parameter (PD, HD, Ps, Pc) which relates to any spectacle wearer and which is different from each first parameter, in the whole of which range it is desired to ensure the feasibility of the pair of spectacles,
c) verifying, on the basis of the shape of the eye wire (11) and for a characteristic number of values of each second parameter (PD, HD, Ps, Pc), that the pair of spectacles is feasible irrespective of the value of each first parameter (PD, HD, Ps, Pc) included in its range so that, when the identified wearer wishes to buy a pair of spectacles based on said spectacle frame (10), it is possible to confirm to him/her if said pair of spectacles is feasible by determining the values of said first and second parameters and by verifying that the value of the first parameter is within said range acquired in step a) and the value of the second parameter is within said range acquired in step b) and is such that the pair of glasses is feasible within the meaning of step c), wherein said first and second parameters (PD, HD, Ps, Pc) are ophthalmic, morphological or geometric-morphological parameters characterizing spectacle wearers,
wherein said first parameters include the spherical refractive power (Ps) and the cylindrical refractive power (Pc), and
wherein said second parameter(s) comprise(s) pupillary height (HD) and/or pupillary half-distance (PD).

2. Testing process according to the preceding claim, wherein said second parameters include pupillary height (HD) and pupillary half-distance (PD).

3. Testing process according to either of the preceding claims, wherein:
- in step a), a range of values of two first parameters (PD, HD, Ps, Pc) is acquired, and
- in step c), it is verified, for a characteristic number of values of each second parameter (PD, HD, Ps, Pc), that the pair of spectacles is feasible irrespective of the values of the two first parameters (PD, HD, Ps, Pc) included in their range.

4. Testing process according to one of the preceding claims, wherein:
- in step b), a range of values of two second parameters (PD, HD, Ps, Pc) is acquired, and
- in step c), it is verified, for a characteristic number of pairs of values of the second parameters, that the pair of spectacles is feasible irrespective of the value of the first parameter (PD, HD, Ps, Pc) included in its range.

5. Testing process according to one of the preceding claims, wherein, in step c), a degree of feasibility of the pair of spectacles is evaluated, said degree of feasibility possibly taking at least three different values.

6. Testing process according to one of the preceding claims, wherein provision is made, prior to step a), for a step of acquiring curvature data of the spectacle frame (10) and wherein, in step a), the range of values of the first parameters is chosen as a function of said curvature data of the spectacle frame (10).

7. Testing process according to one of the preceding claims, wherein, considering a subrange of the range of values of each second parameter (PD, HD, Ps, Pc) wherein the pair of spectacles is not feasible, provision is made for a step consisting in:
d) verifying, for a characteristic number of values of each first parameter (PD, HD, Ps, Pc), that the pair of spectacles is feasible irrespective of the value of each second parameter (PD, HD, Ps, Pc) included in said subrange.

8. Testing process according to one of the preceding claims, wherein, considering a subrange of the range of values of each second parameter (PD, HD, Ps, Pc) wherein the pair of spectacles is feasible and an overrange that encompasses the range of values of the first parameter (PD, HD, Ps, Pc), provision is made for a step consisting in:
d) verifying, for a characteristic number of values of the first parameter (PD, HD, Ps, Pc) within said overrange, that the pair of spectacles is feasible irrespective of the value of each second parameter (PD, HD, Ps, Pc) included in said subrange.

9. Method for classifying spectacle frames (10), by means of a database comprising entries each associated with a spectacle frame, comprising:
- a computing operation during which the testing process according to one of the preceding claims is implemented for an identified spectacle frame (10), and
- a storage operation during which the range of values of each first parameter and also a range of values of each second parameter in the whole of which the pair of spectacles is feasible is recorded in a new entry of the database associated with said identified spectacle frame (10), for the purpose of making several feasible pairs of spectacle frames available to an identified wearer.

10. Classification method according to the preceding claim, wherein provision is made for:
- an operation for acquiring a first particular value of the first parameter and a second particular value of the second parameter, these first and second values corresponding to a given spectacle wearer, and
- an operation for selecting entries of said database wherein the ranges stored comprise said first and second particular values.
